(19) Europäisches Patentamt
European Patent Office
Office européen des brevets

(11) Publication number: **0 215 733**
**B1**

(12) **EUROPEAN PATENT SPECIFICATION**

(45) Date of publication of patent specification: **21.06.89**

(51) Int. Cl.$^4$: **C 07 D 213/64**, C 07 D 413/12, A 01 N 43/40, A 01 N 43/76

(21) Application number: **86810353.2**

(22) Date of filing: **12.08.86**

(54) **Pyridyloxyphenoxypropionic acid derivatives.**

(30) Priority: **19.08.85 US 766730**
**19.08.85 US 766731**

(43) Date of publication of application:
**25.03.87 Bulletin 87/13**

(45) Publication of the grant of the patent:
**21.06.89 Bulletin 89/25**

(84) Designated Contracting States:
**AT BE CH DE FR GB IT LI NL**

(56) References cited:
**EP-A-0 003 313**
**DE-A-2 755 536**
**DE-A-3 247 930**

(73) Proprietor: **SANDOZ AG**
**Lichtstrasse 35**
**CH-4002 Basel (CH)**
(84) **BE CH FR GB IT LI NL**

(73) Proprietor: **SANDOZ-PATENT-GMBH**
**Humboldtstrasse 3**
**D-7850 Lörrach (DE)**
(84) **DE**

(73) Proprietor: **SANDOZ-ERFINDUNGEN**
**Verwaltungsgesellschaft m.b.H.**
**Brunner Strasse 59**
**A-1235 Wien (AT)**
(84) **AT**

(72) Inventor: **Nickell, Louis G.**
**3730 N. Lake Shore Drive**
**Chicago, I11. 60613 (US)**
Inventor: **Stach, Leonard J.**
**274 Southcote**
**Riverside, I11 60546 (US)**
Inventor: **Hokama, Takeo**
**2036 W. Foster**
**Chicago, I11 60625 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 215 733 B1**

## Description

This invention relates to novel 2-[4-(2-pyridyloxy)phenoxy]propionic acid derivatives of the formula (I)

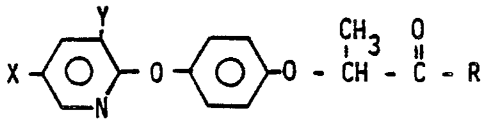

(I)

wherein

X is chlorine or trifluoromethyl,

Y is chlorine or hydrogen,

R is 2-oxazolidinon-3-yl or a group O—CH(Z)—$CH_2$—N($CH_3$)—$SO_2$—$CH_3$ in which Z is hydrogen or methyl.

In addition this invention relates to a method of increasing the yield of sugar obtained from sugarcane and more particularly to a method of increasing the recoverable sugar in sugarcane by treating the sugarcane during its maturation with these novel compounds. The invention also provides plant growth regulating compositions comprising a compound of formula (I).

A variety of plant growth regulators, stimulants and promotors have been tried in the past in attempts to increase the yields of cultivated crops. It has been found that materials that have an effect on one crop will not necessarily have an effect or have a different effect on other crops.

It has now been found that the recovery of sugar from sugarcane can be significantly increased by the use of certain pyridyloxy-phenoxy-propionic acid derivatives. Consequently it has now been found that it is possible to increase the recoverable sugar in sugarcane by contacting the sugarcane plant with an effective amount of the aforedescribed compounds.

The compounds of the present invention can be prepared by reacting an acid chloride of the formula (II)

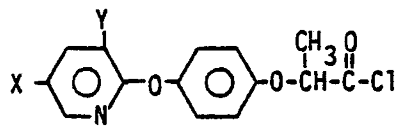

(II)

wherein X and Y are as defined above,
with a compound of formula (III)

H—R (III)

wherein R is as previously defined.

The reaction is conveniently effected in a solvent which is inert under the reaction conditions such as $CH_2Cl_2$, toluene or acetone. A suitable reaction temperature is from 5°C to 60°C. It is in general advantageous to effect the reaction in the presence of an acid scavenger such as triethylamine.

The following examples are provided to illustrate the practice of the invention. Temperature is given in degrees centigrade.

### Example 1

1-(N-Methyl-Methanesulfonamido)-2-Propanol

N-Methylmethanesulfonamide (21.8 g, 0.2 mol), t-butylalcohol (50 ml), sodium hydride (0.5 g, 50% in mineral oil), and propylene oxide (11.6 g, 0.2 mol) were placed into a 3-necked glass reaction flask equipped with stopper, stirrer, thermometer, and reflux condenser. The mixture was heated at 50—60° for three hours, cooled, and concentrated on a rotoevaporator. The concentrate was neutralized with concentrated hydrochloric acid (1 ml) and distilled under reduced pressure. Fractions 4 and 5 yielded the title compound: boiling point (140—142°/1.5 mm Hg).

### Example 2

1-(N-Methyl methanesulfonamido)-prop-2-yl 2-[4-(5-trifluoromethylpyridyl-2-oxy)phenoxy]-propionate

1-N-Methylmethanesulfonamido-2-propanol (40 g, 0.024 mol), triethylamine (5 ml) and dichloromethane (150 ml) were placed into a 3-necked reaction flask equipped with stirrer. The mixture was cooled to 5° and 2-[4-(5-trifluoromethylpyridyl-2-oxy)-phenoxy] propionyl chloride (5.5 g, 0.016 mol) in dichloromethane (30 ml) was added over a 5 minute period. The mixture was then stirred for 1 hour, washed once with water (50 ml), twice with potassium carbonate (50 ml, 10%) passed through phase separating paper and concentrated resulting in a red oil (6.5 g). This concentrate was chromatographed through Florisil clay (150 ml) using hexane-ethyl acetate solvent. Fractions 8—10 yielded a yellow gum (1.2 g) which was recrystallized from ethyl ether to give the title compound as a white solid (0.4 g), melting point 123—125°.

Example 3

1-(N-Methyl methanesulfonamido)-prop-2-yl 2-[4-(5-chloropyridyl-2-oxy)phenoxy]-propionate

1-N-Methylmethanesulfonamido-2-propanol (1.6 g, 0.0096 mol); toluene (80 m) and triethylamine (5 ml) were placed into a 3-necked glass reaction flask equipped with stirrer, thermometer, addition funnel and nitrogen line. 2-[4(5-(Chloropyridyl-2-oxy)phenoxy]propionyl chloride (2.87 g, .0092 mol) in toluene solution was added to the mixture dropwise. Then the mixture was stirred at room temperature for an additional 2.5 hours. At the end of this time period, the reaction mixture was transferred to a separatory funnel, washed four times with water (60 ml), dried through phase separation paper and stripped leaving a red/orange oil (3.5 grams). This oil was chromatographed through Florisil clay using hexane-ethyl acetate solvent. Fractions 9—13 were combined and analysed for the desired product.

| | Theoretical (%) | Found (%) |
|---|---|---|
| Carbon | 51.52 | 49.94 |
| Hydrogen | 5.23 | 5.18 |
| Chlorine | 8.00 | 10.06 |
| Nitrogen | 6.33 | 5.97 |
| Sulfur | 7.24 | 6.79 |

Example 4

2-(N-Methyl methanesulfonamido)-ethyl 2-[4-(5-chloropyridyl-2-oxy)phenoxy]propionate

2-N-Methylmethanesulfonamidoethanol (1.65 g, 0.0107 mol); acetone (80 ml), toluene (30 ml), and triethylamine (5 ml) were placed into a 3-necked glass reaction flask equipped with stirrer, thermometer, addition funnel and nitrogen line. 2-[4-(5-Chloropyridyl-2-oxy)phenoxy]propionyl chloride (3.18 g, 0.0102 mol) in toluene solution was stirred into the mixture dropwise. Then the mixture was stirred at room temperature until the reaction was complete. It was then transferred to a round bottomed flask, stripped of acetone and toluene added. The mixture was washed in a separatory funnel four times with water (60 ml), dried through phase separation paper and stripped of solvent leaving an orange oily gum (4.5 g). This product was chromatographed through Florisil clay (150 ml) with hexane-ethyl acetate solvent mixtures. Fractions 7—10 were combined and analyzed for the desired product as follows:

| | Theoretical (%) | Found (%) |
|---|---|---|
| Carbon | 50.41 | 49.29 |
| Hydrogen | 4.94 | 4.89 |
| Chlorine | 8.27 | 9.54 |
| Nitrogen | 6.53 | 6.19 |
| Sulfur | 7.48 | 7.36 |

Example 5

2-(N-Methyl methanesulfonamido)-ethyl 2-[4-(3,5-dichloropyridyl-2-oxy)phenoxy]propionate

2-N-Methylmethanesulfonamidoethanol (1.65 g, 0.0107 mol), acetone (30 ml), toluene (80 ml) and triethylamine (10 ml) were placed into a 3-necked glass rèaction flask equipped with stirrer, thermometer, addition funnel and nitrogen line. 2-[4-(3,5-Dichloropyridyl-2-oxy)phenoxy]propionyl chloride (3.46 g, 0.01 ml) in toluene solution was stirred into the mixture dropwise. Then the mixture was stirred at room temperature until the reaction was complete, transferred to a round bottom flask, and stripped of acetone. Toluene was added. and the solution washed in a separatory funnel four times with water (60 ml). The product mixture was then dried through phase separation paper and stripped to leave an orange oil/gum (4.6 g). This gum was chromatographed through Florisil clay (150 ml) and eluted with hexane-ethyl acetate solvent mixtures. Fractions 6—8 were consistent in infrared and elemental analysis with the desired product.

| | Theoretical (%) | Found (%) |
|---|---|---|
| Carbon | 46.66 | 45.42 |
| Hydrogen | 4.35 | 4.30 |
| Chlorine | 15.30 | 17.65 |
| Nitrogen | 6.05 | 5.74 |
| Sulfur | 6.92 | 6.51 |

Example 6

1-(N-Methyl methanesulfonamido)prop-2-yl 2-[4-(3,5-Dichloropyridyl-2-oxy)phenoxy]propionate

1-N-Methylmethanesulfonamido-2-propanol (2.0 g, 0.012 mol), triethylamine (3 ml) and acetone (100 ml) were placed into a glass reaction flask equipped with stirrer, dropping funnel, thermometer and reflux condenser and cooled to 5°. 2-[4-(3,5-Dichloropyridyl-2-oxy)phenoxy]propionyl chloride (5.6 g, 0.012 mol) in dichloromethane (20 ml) was added dropwise over a 5 minute period at 5—10°. This mixture was stirred for 1 hour, transferred to a round bottomed flask and stripped on a rotoevaporator. The concentrate was dissolved in toluene (100 ml), and washed with water (100 ml), twice with potassium carbonate solution (100 ml), and water (100 ml). It was dried through phase separating paper and concentrated leaving a product (3.6 g) which was then chromatographed through Florisil clay using hexane-ethyl acetate solvent (150 ml). Fractions 3—6 were analyzed for the desired product as follows:

| | Theoretical (%) | Found (%) |
|---|---|---|
| Carbon | 47.80 | 46.94 |
| Hydrogen | 4.65 | 4.74 |
| Chlorine | 14.85 | 16.19 |
| Nitrogen | 5.87 | 5.45 |
| Sulfur | 6.72 | 6.01 |

Infrared spectrum was consistant with structure.

Example 7

2-(N-Methylmethanesulfonamido)ethyl 2-[4-(5-trifluoromethylpyridyl-2-oxy)phenoxy]propionate

2-N-Methylmethanesulfonamidoethanol (3.1 g, 0.02 mol), triethylamine (5 ml) and dichloromethane (150 ml) were placed into a glass reaction flask equipped with a dropping funnel, stirrer, thermometer and nitrogen inlet tube. 2-[4-(5-Trifluoromethylpyridyl-2-oxy)phenoxy]propionyl chloride (5.5 g, 0.015 mol) dissolved in dichloromethane (30 ml) was added dropwise over a five minute period at 5—10°. The mixture was stirred at room temperature for 2 hours, transferred to a separatory funnel and washed twice with water (50 ml) and twice with potassium carbonate (50 ml).

It was then passed through phase separating paper and concentrated on a rotoevaporator leaving a red oil (6.5 g). The oil was chromatographed through Florisil clay (150 ml) using hexane-ethyl acetate solvent and Fractions 10—12 analyzed for the desired product as follows:

| | Theoretical (%) | Found (%) |
|---|---|---|
| Carbon | 49.35 | 49.32 |
| Hydrogen | 4.58 | 4.61 |
| Nitrogen | 6.06 | 5.98 |
| Sulfur | 6.93 | 7.26 |

Example 8

2-[4-(5-trifluoromethyl-2-pyridinyloxy)phenoxy]propionyl-2-oxazolidinone

2-Oxazolidone (1.5 g, 0.017 mol), dichloromethane (90 ml), and triethylamine (5 ml) were placed into a 3-necked glass reaction flask equipped with stirrer, reflux condenser and thermometer. This mixture was cooled to 10°. 2-[4-(5-Trifluorometyl-2-pyridinyloxy)phenoxy]propionic acid chloride (5.7 g, 0.016 mol) in dichloromethane (30 ml) was added to the cooled mixture which was then stirred for 3 hours at room temperature. This product mixture was washed with water (60 ml) and (5%) sodium bicarbonate (70 ml) and concentrated to yield an orange gum (6.9 g) which was dissolved in hot toluene, treated with charcoal, filtered, and concentrated. The residue was recrystallized from a mixture of dichloromethane and hexane leaving a white solid (3.3 g), melting point 101—102°. Elemental analysis for the desired product was as follows:

| | Theoretical (%) | Found (%) |
|---|---|---|
| Carbon | 54.55 | 54.56 |
| Hydrogen | 3.82 | 3.88 |
| Nitrogen | 7.07 | 6.98 |

Infrared analysis was consistent with the product, 2-[4-5-trifluoromethyl-2-pyridinyloxy)phenoxy]propionyl-2-oxazolidinone.

Biological Effectiveness

The effectiveness of the compounds for increasing the recoverable sugar in sugarcane is demonstrated by the following three tests. The cane was harvested eight weeks after application of the compound tested.

The top 14 joints of the treated cane was as well as those of the controls were removed, combined and analyzed for juice purity and pol percent cane, following the "press method" developed and described by T. Tanimoto, Hawaiian Planters Record, 57, 133 (1964). Pol percent cane is a polarimetric determination and equals the percentage of sucrose if the latter is the only substance in the solution which will rotate the plane of polarized light. The pol percent cane is a standard method of determining the sucrose content of sugarcane.

TABLE A

| | Rate of Application (lbs./acre) | Pol % Cane | Juice Purity |
|---|---|---|---|
| **TEST 1** | | | |
| Compound of Example 2 | 1 | 13.00 | 82.87 |
| Control | 0 | 7.54 | 68.88 |
| **TEST 2** | | | |
| Compound of Example 2 | 1 | 10.71 | 76.61 |
| Control | 0 | 8.54 | 69.31 |
| **TEST 3** | | | |
| Compound of Example 2 | 1 | 14.53 | 88.71 |
| | 0.5 | 13.07 | 86.18 |
| | 0.1 | 13.49 | 87.60 |
| Control | 0 | 10.78 | 81.76 |

TABLE 8

| | Rate of Application (lbs./acre) | Pol % Cane | Juice Purity |
|---|---|---|---|
| | TEST 1 | | |
| Compound of Example 8 | 1 | 15.12 | 86.83 |
| Control | 0 | 10.06 | 76.54 |
| | TEST 2 | | |
| Compound of Example 8 | 1 | 14.91 | 85.91 |
| Control | 0 | 11.61 | 80.66 |
| | TEST 3 | | |
| Compound of | 1 | 12.51 | 83.57 |
| Example 8 | 0.5 | 12.21 | 83.66 |
| Control | 0 | 10.78 | 81.76 |

In the use of the compounds of the invention to increase the recoverable sugar in sugarcane, sugarcane is treated at a late stage of development of the sugarcane wherein most of the sugar formation takes place. Thus, under normal growing conditions and common cultivation practices the active compound of this invention can be applied to the sugarcane during the period of from about 2 to about 10 weeks before harvesting.

The amount of active compound required to effectively increase the recoverable sugar from sugarcane can vary somewhat depending on such factors as the particular compound employed, the time of application, the weather, crop density, method of application and the like. Generally, an amount of at least 0.1 pounds per acre and preferably an amount of from 0.1 pounds per acre to about 10 pounds per acre can be used.

For practical use in treating sugarcane, the active compounds of this invention are generally incorporated into compositions which comprise an inert diluent and an effective amount of the compound. The compositions enable the active compound to be conveniently applied to the sugarcane at the desired rate.

They may be employed in either solid or liquid forms e.g. in the form of a wettable powder or an emulsifiable concentrate incorporating conventional diluents. Such compositions may be produced in conventional manner, e.g. by mixing the active ingredient with a diluent and optionally other formulating ingredients such as surfactants.

The term diluents as used herein means any liquid or solid agriculturally acceptable material which may be added to the active constituent to bring it in an easier or improved applicable form, respectively to a usable or desirable strength of activity. It can for example be talc, kaolin, diatomaceous earth, mineral oil or water.

Particularly formulations to be applied in spraying forms such as water dispersible concentrates, wettable powders or emulsifiable concentrates may contain surfactants such as wetting and dispersing agents, e.g. the condensation product of formaldehyde with naphthalene sulphonate, an alkylarylsulphonate, a lignin sulphonate, a fatty alkyl sulphate, an ethoxylated alkylphenol and an ethoxylated fatty alcohol.

In general, the formulations include from 0.01 to 90% by weight of active agent from 0 to 20% by weight of agriculturally acceptable surfactant and 99.99 to 10% by weight (solid or liquid) diluent(s).

Concentrate forms of compositions generally contain between about 2 and 90%, preferably between

about 5 and 70% by weight of active agent. Application forms of formulation may for example contain from 0.01 to 20% by weight of active agent.

Typical formulations according to the present invention useful for increasing the recoverable sugar in sugarcane are illustrated in the following examples wherein the quantities are given in parts by weight.

Composition Example 1

Emulsifiable Concentrate

The following ingredients are blended thoroughly until a homogeneous liquid concentrate is obtained. This concentrate is mixed with water to give an aqueous dispersion containing the desired concentration of the active ingredients for use as a spray.

| | |
|---|---|
| Product of Example 2 or 8 | 25 |
| Sodium lauryl sulfate | 2 |
| Sodium lignin sulfate | 3 |
| Kerosene | 70 |

Composition Example 2

Wettable Powder

The following components are mixed intimately in conventional mixing or blending equipment and are then ground to a powder having a particle size of less than about 50 microns. The finished powder is dispersed in water to give the desired concentration of active compound for application to the sugarcane.

| | |
|---|---|
| Product of Example 3 or 8 | 50 |
| Fuller's earth | 47 |
| Sodium lauryl sulfate | 2.5 |
| Methyl cellulose | 0.5 |

Composition Example 3

Dust

The following ingredients are mixed thoroughly and are then ground to an average particle size of less than about 50 microns to give a dust suitable for application with conventional dusting equipment.

| | |
|---|---|
| Product of Example 4 or 8 | 10 |
| Powdered talc | 90 |

**Claims for the Contracting States: BE CH DE FR GB IT LI NL**

1. A compound of the formula (I)

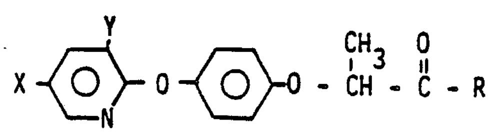

(I)

wherein

X is chlorine or trifluoromethyl,

Y is chlorine or hydrogen,

R is 2-oxazolidinon-3-yl or a group $O—CH(Z)—CH_2—N(CH_3)—SO_2—CH_3$ in which Z is hydrogen or methyl.

2. The compound of claim 1, 1-(N-methylmethanesulfonamido)-prop-2-yl 2-[4-(5-trifluoromethylpyridyl-2-oxy)-phenoxy]propionate.

3. The compound of claim 1, 1-(N-methylmethanesulfonamido)-prop-2-yl 2-[4-(5-chloropyridyl-2-oxy)-phenoxy]propionate.

4. The compound of claim 1, 2-(N-methylmethanesulfonamido)-ethyl 2-[4-(3,5-chloropyridyl-2-oxy)-phenoxy]propionate.

5. The compound of claim 1, 2-(N-methylmethanesulfonamido)-ethyl 2-[4-(3,5-dichloropyridyl-2-oxy)-phenoxy]propionate.

6. The compound of claim 1, 1-(N-methylmethanesulfonamido)prop-2-yl 2-[4-(3,5-dichloropyridyl-2-oxy)phenoxy]propionate.

7. The compound of claim 1, 2-(N-methylmethanesulfonamido)ethyl 2-[4-(5-trifluoromethylpyridyl-2-oxy)phenoxy]propionate.

8. The compound of claim 1, 2-[4-(5-trifluoromethyl-2-pyridinyloxy)phenoxy]propionyl-2-oxazolidinone.

9. A method for increasing the recoverable sugar contained in sugarcane which comprises contacting the sugarcane plant with an effective amount of a compound of claim 1 to 8.

10. Process of preparing a compound of formula (I) stated in claim 1, which comprises reacting an acid chloride of the formula (II)

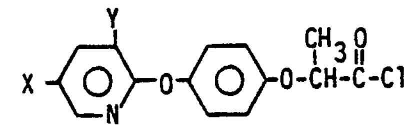

(II)

wherein X and Y are as stated in claim 1, with a compound of formula (III)

H—R (III)

wherein R is as stated in claim 1.

11. A plant growth regulating composition comprising a compound according to any one of claims 1 to 8 and a diluent.

**Claims for the Contracting State: AT**

1. A plant growth regulating composition comprising a compound of formula (I)

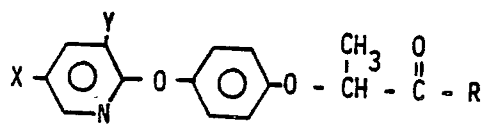

(I)

wherein

X is chlorine or trifluoromethyl,

Y is chlorine or hydrogen,

R is 2-oxazolidinon-3-yl or a group $O—CH(Z)—CH_2—N(CH_3)—SO_2—CH_3$ in which Z is hydrogen or methyl.

2. The composition of claim 1, comprising 1-(N-methylmethanesulfonamido)-prop-2-yl 2-[4-(5-trifluoromethylpyridyl-2-oxy)-phenoxy]propionate.

3. The composition of claim 1, comprising 1-(N-methylmethanesulfonamido)-prop-2-yl 2-[4-(5-chloropyridyl-2-oxy)phenoxy]propionate.

4. The composition of claim 1, comprising 2-(N-methylmethanesulfonamido)-ethyl 2-[4-(5-chloropyridyl-2-oxy)phenoxy]propionate.

5. The composition of claim 1, comprising 2-(N-methylmethanesulfonamido)-ethyl 2-[4-(3,5-dichloropyridyl-2-oxy)phenoxy]propionate.

6. The composition of claim 1, comprising 1-(N-methylmethanesulfonamido)prop-2-yl 2-[4-(3,5-dichloropyridyl-2-oxy)phenoxy]propionate.

7. The composition of claim 1, comprising 2-(N-methylmethanesulfonamido)ethyl 2-[4-(5-trifluoromethylpyridyl-2-oxy)phenoxy]propionate.

8. The composition of claim 1, comprising 2-[4-(5-trifluoromethyl-2-pyridinyloxy)phenoxy]propionyl-2-oxazolidinone.

9. Process for preparing a compound of formula (I) stated in any one of claims 1 to 8, which comprises reacting an acid chloride of the formula (II)

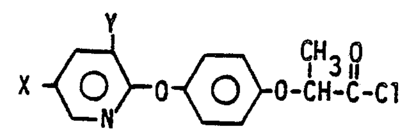

(II)

wherein X and Y are as stated in claim 1, with a compound of formula (III)

H—R (III)

wherein R is as stated in claim 1.

**Patentansprüche für die Vertragsstaaten: BE CH DE FR GB IT LI NL**

1. Eine Verbindung der Formel

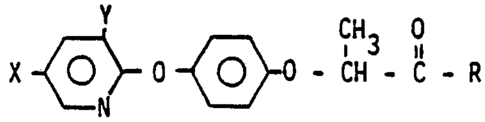 (I)

worin

X für Chlor oder Trifluormethyl steht,

Y für Chlor oder Wasserstoff steht,

R für 2-Oxazolidinon-3-yl oder eine Gruppe $O—CH(Z)—CH_2—N(CH_3)—SO_2—CH_3$ steht,

worin Z Wasserstoff oder Methyl bedeuten.

2. Die Verbindung gemäss Anspruch 1, 1-(N-Methylmethansulfonamido)-prop-2-yl 2-[4-(5-Trifluormethylpyridyl-2-oxy)-phenoxy]propionat.

3. Die Verbindung gemäss Anspruch 1, 1-(N-Methylmethansulfonamido)-prop-2-yl 2-[4-(5-Chlorpyridyl-2-oxy)phenoxy]propionat.

4. Die Verbindung gemäss Anspruch 1, 2-(N-Methylmethansulfonamido)-ethyl 2-[4-(3,5-Chlorpyridyl-2-oxy)phenoxy]propionat.

5. Die Verbindung gemäss Anspruch 1, 2-(N-Methylmethansulfonamido)-ethyl 2-[4-(3,5-Dichlorpyridyl-2-oxy)phenoxy]propionat.

6. Die Verbindung gemäss Anspruch 1, 1-(N-Methylmethansulfonamido)prop-2-yl 2-[4-(3,5-Dichlorpyridyl-2-oxy)phenoxy]propionat.

7. Die Verbindung gemäss Anspruch 1, 2-(N-Methylmethansulfonamido)ethyl 2-[4-(5-Trifluormethylpyridyl-2-oxy)phenoxy]propionat.

8. Die Verbindung gemäss Anspruch 1, 2-[4-(5-Trifluormethyl-2-pyridinyloxy)phenoxy] propionyl-2-oxazolidinon.

9. Ein Verfahren zur Erhöhung des zurückgewinnbaren Zuckers in Zuckerrohr, dadurch gekennzeichnet, dass man die Zuckerrohrpflanze mit einer wirksamen Menge einer Verbindung gemäss Anspruch 1 bis 8 in Kontakt bringt.

10. Verfahren zur Herstellung einer Verbindung der Formel (I) definiert in Anspruch 1, dadurch gekennzeichnet, dass man ein Säurechlorid der Formel (II)

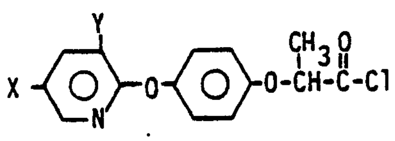 (II)

worin X und Y die in Anspruch 1 angegebene Bedeutung besitzen, mit einer Verbindung der Formel (III)

$$H—R \qquad (III)$$

worin R die in Anspruch 1 angegebene Bedeutung besitzt, umsetzt.

11. Ein das Pflanzenwachstum regulierende Zubereitung enthaltend eine Verbindung gemäss Ansprüche 1 bis 8 und einen Verdünner.

**Patentansprüche für den Vertragsstaat: AT**

1. Eine das Pflanzenwachstum regulierende Zubereitung enthaltend eine Verbindung der Formel (I)

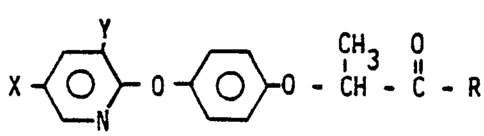 (I)

worin

X für Chlor oder Trifluormethyl steht,

Y für Chlor oder Wasserstoff steht,

R für 2-Oxazolidinon-3-yl oder eine Gruppe $O—CH(Z)—CH_2—N(CH_3)—SO_2—CH_3$ steht,

worin Z Wasserstoff oder Methyl bedeutet.

2. Die Zubereitung gemäss Anspruch 1, enthaltend 1-(N-Methylmethansulfonamido)-prop-2-yl 2-[4-(5-Trifluormethylpyridyl-2-oxy)-phenoxy]propionat.

3. Die Zubereitung gemäss Anspruch 1, enthaltend 1-N-Methylmethansulfonamido)-prop-2-yl 2-[4-Chlorpyridyl-2-oxy)phenoxy]propionat.

4. Die Zubereitung gemäss Anspruch 1, enthaltend 2-(N-Methylmethansulfonamido)ethyl 2-[4-(3,5-Dichlorpyridyl-2-oxy)phenoxy]propionat.

5. Die Zubereitung gemäss Anspruch 1, enthaltend 2-(N-Methylmethansulfonamido)ethyl 2-[4-(3,5-Dichlorpyridyl-2-oxy)phenoxy]propionat.

6. Die Zubereitung gemäss Anspruch 1, enthaltend 1-(N-Methylmethansulfonamido)-prop-2-yl 2-[4-(3,5-Dichlorpyridyl-2-oxy)phenoxy]propionat.

7. Die Zubereitung gemäss Anspruch 1, enthaltend 2-(N-Methylmethansulfonamido)ethyl 2-[4-(5-Trifluormethylpyridyl-2-oxy)phenoxy]propionat.

8. Die Zubereitung gemäss Anspruch 1, enthaltend 2-[4-(5-Trifluormethyl-2-pyridinyloxy)phenoxy]propionyl-2-oxazolidinon.

9. Verfahren zur Herstellung einer Verbindung der Formel (I), definiert in den Ansprüchen 1 bis 8, dadurch gekennzeichnet, dass man ein Säurechlorid der Formel II

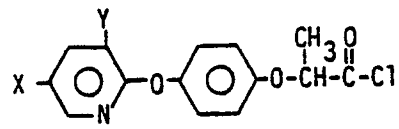

(II)

worin X und Y die in Anspruch 1 angegebene Bedeutung besitzen, mit einer Verbindung der Formel (III)

H—R (III)

worin R die in Anspruch 1 angegebene Bedeutung besitzt, umsetzt.

**Revendications pour les Etats contractants: BE CH DE FR GB IT LI NL**

1. Un composé de formule I

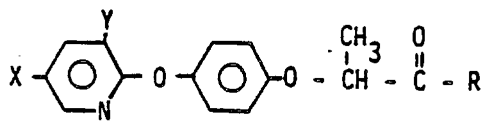

(I)

dans laquelle

X signifie le chlore ou un groupe trifluorométhyle,

Y signifie le chlore ou l'hydrogène,

R signifie un groupe 2-oxazolidinone-3-yle ou un groupe O—CH(Z)—$CH_2$—N($CH_3$)—$SO_2$—$CH_3$ dans lequel Z signifie l'hydrogène ou un groupe méthyle.

2. Le composé de la revendication 1, le 2-[4-(5-trifluorométhylpyridyl-2-oxy)-phénoxy]propionate de 1-(N-méthylméthanesulfonamido)-prop-2-yle.

3. Le composé de la revendication 1, le 2-[4-(5-chloropyridyl-2-oxy)phenoxy]propionate de 1-(N-méthylméthanesulfonamido)-prop-2-yle.

4. Le composé de la revendication 1, le 2-[4-(3,5-dichloropyridyl-2-oxy)phénoxy]propionate de 2-(N-méthylméthanesulfonamido)-éthyle.

5. Le composé de la revendication 1, le 2-[4-(3,5-dichloropyridyl-2-oxy)phénoxy]propionate de 2-(N-méthylméthanesulfonamido)-éthyle.

6. Le composé de la revendication 1, le 2-[4-(3,5-dichloropyridyl-2-oxy)-phénoxy]-propionate de 1-(N-méthylméthanesulfonamido)prop-2-yle.

7. Le composé de la revendication 1, le 2-[4-(5-trifluorométhylpyridyl-2-oxy)phénoxy]propionate de 2-(N-méthylméthanesulfonamido)éthyle.

8. Le composé de la revendication 1, la 2-[4-(5-trifluorométhyl-2-pyridinyloxy)phénoxy]propionyl-2-oxazolidinone.

9. Une méthode pour augmenter le sucre récupérable contenu dans la canne à sucre qui comprend la mise en contact de la plante de canne à sucre avec une quantité efficace d'un composé des revendications 1 à 8.

10. Procédé de préparation d'un composé de formule I spécifié à la revendication 1, qui comprend la réaction d'un chlorure d'acide de formule II

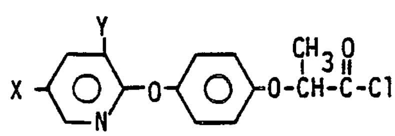

(II)

dans laquelle X et Y sont tels que spécifiés à la revendication 1, avec un composé de formule III

H—R (III)

dans laquelle R est tel que spécifié à la revendication 1.

11. Une composition régulatrice de la croissance des plantes comprenant un composé selon l'une quelconque des revendications 1 à 8 et un diluant.

**Revendications pour l'Etat contractant: AT**

1. Une composition régulatrice de la croissance des plantes comprenant un composé de formule I

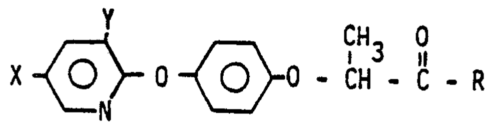

(I)

dans laquelle

X signifie le chlore ou un groupe trifluorométhyle,

Y signifie le chlore ou l'hydrogène,

R signifie un groupe 2-oxazolidinone-3-yle ou un groupe O—CH(Z)—$CH_2$—N($CH_3$)—$SO_2$—$CH_3$ dans lequel Z signifie l'hydrogène ou un groupe méthyle.

2. La composition de la revendication 1, comprenant le 2-[4-(5-trifluorométhylpyridyl-2-oxy)-phénoxy]-propionate de 1-(N-méthylméthanesulfonamido)-prop-2-yle.

3. La composition de la revendication 1, comprenant le 2-[4-(5-chloropyridyl-2-oxy)phenoxy]propionate de 1-(N-méthylméthanesulfonamido)-prop-2-yle.

4. La composition de la revendication 1, comprenant le 2-[4-(3,5-dichloropyridyl-2-oxy)phénoxy]propionate de 2-(N-méthylméthanesulfonamido)-éthyle.

5. La composition de la revendication 1, comprenant le 2-[4-(3,5-dichloropyridyl-2-oxy)phénoxy]propionate de 2-(N-méthylméthanesulfonamido)-éthyle.

6. La composition de la revendication 1, comprenant le 2-[4-(3,5-dichloropyridyl-2-oxy)-phénoxy]-propionate de 1-(N-méthylméthanesulfonamido)prop-2-yle.

7. La composition de la revendication 1, comprenant le 2-[4-(5-trifluorométhylpyridyl-2-oxy)phénoxy]-propionate de 2-(N-méthylméthanesulfonamido)éthyle.

8. La composition de la revendication 1, comprenant la 2-[4-(5-trifluorométhyl-2-pyridinyloxy)phénoxy]propionyl-2-oxazolidinone.

9. Procédé de préparation d'un composé de formule I spécifié à l'une quelconque des revendications 1 à 8, qui comprend la réaction d'un chlorure d'acide de formule II

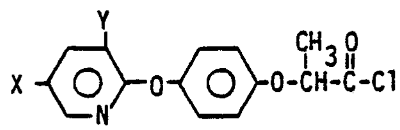

(II)

dans laquelle X et Y sont tels que spécifiés à la revendication 1, avec un composé de formule III

H—R (III)

dans laquelle R est tel que spécifié à la revendication 1.